# EUROPEAN PATENT SPECIFICATION

(11) **EP 2 510 126 B1**
(45) Date of publication and mention of the grant of the patent: **09.08.2017**
(21) Application number: 10836550.3
(22) Date of filing: 07.12.2010
(51) Int. Cl.: C12Q 1/68, C12N 15/11, G01N 33/50

(54) **MULTI-SAMPLE INDEXING FOR MULTIPLEX GENOTYPING**
INDEXIERUNG MEHRERER PROBEN FÜR MULTIPLEX-GENOTYPISIERUNG
INDEXAGE MULTI-ÉCHANTILLON POUR UN GÉNOTYPAGE MULTIPLEXE

(30) Priority: 07.12.2009 US 267363 P
(43) Date of publication of application: 17.10.2012
(73) Proprietor: Illumina, Inc., San Diego, CA 92122 (US)
(72) Inventor: FAN, Jian-Bing, San Diego California 92130 (US); GUNDERSON, Kevin, Encinitas California 92024 (US)
(74) Representative: Cooley (UK) LLP
(86) International application number: PCT/US2010/059286
(87) International publication number: WO 2011/071923

(56) References cited:
- WO-A1-96/15271
- WO-A1-2007/091077
- WO-A1-2007/100243
- US-A- 6 027 889
- US-A1- 2003 003 490
- US-A1- 2003 108 900
- US-A1- 2004 121 364
- US-B1- 6 355 431
- PARAMESWARAN POORNIMA ET AL: "A pyrosequencing-tailored nucleotide barcode design unveils opportunities for large-scale sample multiplexing", NUCLEIC ACIDS RESEARCH, OXFORD UNIVERSITY PRESS, SURREY, GB, vol. 35, no. 19, 11 October 2007 (2007-10-11), pages e130.1-e130.9, XP002482387, ISSN: 0305-1048, DOI: 10.1093/NAR/GKM760 [retrieved on 2007-10-11]

## Description

### Background of the Invention

The present invention relates to molecular biology, and more specifically to detecting numerous nucleotide sequences of interest in several samples. Multiplex assays may detect numerous sequences in single samples. When numerous samples are involved, however, performing multiplex assays can be labor-intensive and expensive. Thus, there is a need for detection methods that can detect numerous sequences in each of several individual samples, report the presence or absence of each sequence of interest, and correlate this result with the identity of the individual sample.

### Summary of the Invention

The present invention provides methods for determining the presence of a plurality of nucleotide sequences of interest in a plurality of samples, while preserving the identity of each sample. The method can be used in many applications, including genotyping, expression analysis, and identification of individual species in complex samples.

Specifically, the invention provides a method for determining the presence of a plurality of nucleotide sequences of interest in a plurality of samples while preserving the identity of each sample, comprising the steps of:
(a) contacting each sample with a plurality of probe sets, each probe set comprising:
   a first probe having a first identification sequence and a first hybridization sequence complementary to a first portion of a sequence of interest, and
   a second probe having a second identification sequence and a second hybridization sequence complementary to a second portion of the same sequence of interest;
   wherein each of the first and the second identification sequence comprises a separate subsequence for identifying the sample and a separate subsequence for identifying the sequence of interest;
(b) joining the first and second probes of a probe set if the first hybridization sequence is hybridized to the first portion of the sequence of interest, and the second hybridization sequence is hybridized to the second portion of the same sequence of interest;
(c) decoding the identification sequences for a plurality of joined probes, wherein decoding the identification sequences comprises paired-end sequencing of the first and second identification sequences and whereby the identity of each sample is preserved; and
(d) determining the presence of the sequence of interest and the identity of the sample containing the sequence of interest based on identification sequence codes present in each joined probe, wherein said determining comprises an error detection step of:
   (i) detecting joint probes having mismatched or unexpected combinations of first and second identification sequence codes; and
   (ii) discarding the information obtained from those joined probes having mismatched or unexpected combinations of first and second identification sequence codes.

### Brief Description of the Drawings

The figures are intended to illustrate broad concepts of the invention using examples in schematic form for ease of explanation to ordinarily skilled persons. They are not intended to limit the scope of the invention to representative embodiments or by showing or omitting optional features of the invention.
**Fig. 1** shows representative ASO1 and LSO probes, where an ASO1 and LSO form a single probe set. For the first sample (sample ID 1), a probe set is provided for each SNP of interest, e.g. SNPs 1, 2, 3,... 1024, hence a pool of probe sets is provided for sample ID 1. Another pool of probe sets is provided for sample ID 2, etc. In this and subsequent figures, the nucleotide sequences of various ID sequences are represented by unique 4-digit numbers.
**Fig. 2** shows representative ASO2 probes that can optionally be used with the probe sets in Fig. 1.
**Fig. 3a** illustrates hybridization of an ASO1 probe and an LSO probe to a sequence of interest that contains a first SNP allele, such as allele T ("SNP1/allele T"), which is present in the genomic DNA in sample 1. **Fig. 3b** shows an alternate hybridization of an ASO2 probe (and an LSO probe) that occurs if sample 1 contains SNP1/allele C instead.
**Fig. 4a** and **Fig. 4b** illustrate hybridization complexes resulting from the hybridizations in Fig. 3a and Fig. 3b, respectively.
**Fig. 5a** and **Fig. 5b** illustrates the two joined probes obtained from the hybridization complexes in Fig. 4a and Fig. 4b, respectively. **Fig. 5c** shows oligonucleotide primers **(1a', 2a', 3a')** that can be used with the primer sequences of the probes to amplify joined probes.
**Fig. 6** illustrates decoding of a joined probe, where the identification sequences provide the sample ID, SNP ID, and allele information.
**Fig. 7** illustrates genotyping using reversible terminators in an SBE reaction, followed by ligation to make a joined probe for sequencing. Optional steps are bracketed.
**Fig. 8** illustrates a variation of the method in **Fig. 7****,** where the "joined probe" is formed by circularizing a single probe.

### Detailed Description

### Samples

The present invention provides methods for determining the presence or absence of nucleotide sequences of interest in samples. In other words, a sample used in the method can contain nucleic acids having particular nucleotide sequences. In one aspect, the method can determine whether these nucleotide sequences contain or do not contain a predetermined nucleotide sequence of interest, such as genotyping alleles of a polymorphism. In another aspect, the method can measure different levels of particular nucleotide sequences, such as for gene expression analysis. In yet another aspect, the method can detect the presence of species- or class-specific sequences to detect individual species or members of a class from a complex sample.

As used herein, the term "sample" refers to a quantity of matter taken from a source, whether comprising the entire source or a part of it, such as a representative portion. The sample can be taken from a single organism, such as a human, a nonhuman animal, a plant, or a microorganism, or can be taken from a mixture of different organisms, such as an environmental source. The origin of the sample can be from mammalian or avian livestock, or from other agricultural sources or produce, such as from seeds, shoots and leaves, or from roots and tubers. The sample can also contain material exogenous to the intended sample source, such as a bacterial or viral pathogen.

A "plurality of samples" then refers to two or more samples, such as from multiple different organisms, including multiple species, or from multiple environmental sources. A plurality of samples can also comprise multiple samples from a single source, such as from different tissues, organs, or from different draws, time points, or different sample preparations or treatments. The invention provides methods that can be applied to 2, 4, 8, 12, 16, 20, 32, 48, 96, 128, 384, 1024, 2048, 4096, 8192, 16,384, or more than 36,864 samples. Such samples will usually contain some nucleic acids, and may be prepared to preserve, enrich, or purify the nucleic acids, according to the particular application.

Each sample can be said to have a unique "identity" where an informational identifier is used to designate a particular sample and to be able to report information related to that particular sample alone, and not to other samples that may be involved in the same or other experiments. This allows detection results obtained by the method to be traced back to a particular sample, even when the physical sample (or its derivatives) is combined or mixed with other samples (and their respective derivatives) at some point and no longer physically separated or distinguishable. Thus, the identity of a sample is "preserved" when information about the properties of the sample can be traced back to the original physical sample, despite physical or informational intermixing with other samples.

The term "nucleic acid" refers to a natural, synthetic, or artificial polynucleotide, such as DNA or RNA, which embodies a sequence of nucleotides. The nucleic acid can be fragmented, cloned, replicated, amplified, or otherwise derived or manipulated. Exemplary DNA species include genomic DNA (gDNA), mitochondrial DNA, and complementary DNA (cDNA). Exemplary RNA species include messenger RNA (mRNA), transfer RNA (tRNA), microRNA (miRNA), small interfering RNA (siRNA), and ribosomal RNA (rRNA).

The methods described herein are applicable to nucleic acids containing complete genomes, substantially complete, representative genomes, representations having substantially full genomic complexity, or reduced complexity samples, such as where certain sequences or classes of sequences are preferentially enriched or represented. In some aspects, the complexity of the nucleic acids is less than half of an original genome.

It can be useful to immobilize a nucleic acid to a bead, to a surface, such as a flow-cell interior surface, or other solid-phase substrate. Examples of immobilization methods include covalent, ionic, affinity and metal-chelation bonding. When beads are used, they may be magnetic, latex, or present streptavidin or biotin moieties. The nucleic acid can be attached to such solid supports in a number of ways.

It can also be useful to attach a purification tag to a nucleic acid. By "purification tag" herein is meant a moiety that can be used to purify a strand of nucleic acid, usually via attachment to a solid support. Suitable purification tags include members of binding partner pairs. For example, the tag may be a hapten or antigen, which will bind its binding partner. In a preferred embodiment, the binding partner can be attached to a solid support. For example, suitable binding partner pairs include antigens (such as proteins or peptides) and antibodies (including fragments thereof, e.g. Fabs); proteins and small molecules, including biotin/streptavidin; enzymes and substrates or inhibitors; other protein-protein interacting pairs; receptor-ligands; and carbohydrates and their binding partners. Pairs of a nucleic acid and a nucleic-acid-binding protein are also useful. In one embodiment, the smaller molecule of the pair is attached to a nucleotide triphosphate (NTP) for incorporation into a probe or primer. Preferred binding partner pairs include biotin (or imino-biotin) and streptavidin, digeoxinin and antibodies.

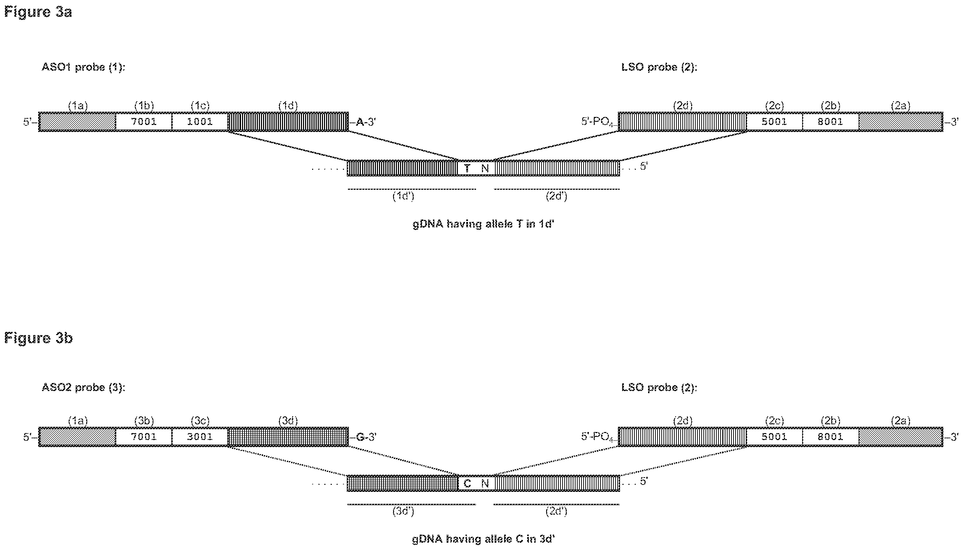

The term "nucleotide sequence of interest" (e.g. **1d'-2d'** or **3d**'-**2d**') can refer to two or more contiguous nucleotides, where the presence or absence of the nucleotides is of interest to the investigator. In one aspect, the sequence of interest comprises a single nucleotide query, such as the site of a single nucleotide polymorphism (SNP), or methylation polymorphism at a single nucleotide site. The polymorphism can also involve more than a single nucleotide, as in the case of an insertion, deletion or transposition. A sequence of interest can also be a nucleotide sequence that is repeated within the nucleic acids of the sample or present in one or a number of copies in the sample. As illustrated in **Fig. 3a****,** the sequence of interest is illustrated by **1d'-2d',** which comprises the SNP allele T. **Fig. 3b** shows an alternate allele C for the same SNP. In this illustration, the N refers to any nucleotide, although in other embodiments, N can be two or more nucleotides, or represent a break in the sugar-phosphate backbone.

In other aspects, the nucleotide sequence of interest is a sequence that is characteristic of a species or a group of species. For example, in a sample of gut flora, a sequence of interest can be unique to a single species of microbes or bacteria, or to members of a class of species, such as a genus-type classification or a functional class as in a defined class of pathogens. Such sequences include rRNA sequences, for instance bacterial 16S rRNA or other sequences that are well-characterized across a wide range of species.

The methods of the present invention can be used in the simultaneous detection of a plurality of nucleotide sequences of interest, such as 2, 4, 8, 16, 32, 48, 64, 96, 128, 192, 384, 1024 or more sequences in a single assay. Such nucleotide sequences of interest are typically preselected by the investigator for inquiry, and may be associated with a particular genotype or phenotype of interest, or may be obtained without such prior information, such as from a random set. For ease of discussion, however, the examples are shown assume a SNP polymorphism, while the ordinarily skilled person would understand that these examples apply to more complex polymorphisms as well.

For purposes of easier description, a nucleotide sequence of interest can be described as having a "first portion" and "second portion", where a polymorphism site is present in either or both portions. For example, **Fig. 3a** shows a sequence of interest **(1d**'-**2d**') present on a gDNA sample, where the sequence has a portion **1d'** and a portion **2d**'. In this particular example, the sequence of interest contains a SNP polymorphism site with allele T at the 5' end of **1d'.** For comparison, a similar sequence of interest (**3d**'-**2d**') is identical to **1d'-2d',** but has a C allele at the same SNP polymorphism site. The portions can be contiguous or noncontiguous, having 1, 2, 3, or more nucleotides identified between the two identified portions. For example, a nucleotide sequence of interest "ABCDEFGHIJKLM" can have a first portion "ABCDEF" and a second portion "IJKLM", where E, F, G, H, I or J may be a SNP or methylation site of interest.

By determining "the presence" of a nucleotide sequence of interest is meant that the method determines whether or not the sequence of interest is present in detectable amounts among the nucleic acids of the sample. For example, when the sequence of interest contains SNP site with a T/C polymorphism, the sample DNA may contain the T allele or the C allele. In other cases, neither allele is present, or both alleles may be present in varying amounts, as with a heterozygous haplotype. The determination can also be used to determine the copy number of a sequence of interest. In gene expression applications, the term extends to determining the level of sequence present, whether in absolute amounts or quantities relative to other samples.

### Probes

The invention achieves its determinations in part by the use of oligonucleotide probes (e.g. **1, 2)** that are specialized for this purpose. As a convention for discussion, a first probe is designated "ASO1" **(1),** a second probe is designated "LSO" **(2).** The probes contain regions that contain or complement the sequence of interest (**1d**, **2d),** such as a specific allele of a polymorphism. However, these terms should not be interpreted as limiting the method to determination of different alleles, but can be applied more generally to any sequence of interest, such as a sequence characteristic of a species or genus of interest. The probes can also contain primer sequences **(1a, 2a)** for use with complementary primers **(1a', 2a'),** as explained further below. In addition, the probes contain specific identification sequences (e.g. **1b, 1c, 2c, 2b)** that, in combination with other information, can provide the identity of a particular sample, sequence of interest, or allele, for example.

The method of the invention also provides an optional "alternate probe", i.e. third probe, for a given probe set, sometimes termed an "ASO2" probe, which is similar to either the first or second probe, but is directed to an alternate allele at the same sequence of interest. As illustrated in **Fig. 3****,** the ASO2 is similar to the ASO1, and but has an alternate hybridization sequence **(3d)** that is complementary or substantially complementary to a different allele of the same sequence of interest as **(2d).**

The term "probe" refers to a single-stranded nucleic acid capable of hybridizing to another single-stranded nucleic acid that has a complementary or substantially complementary nucleotide sequence, under conditions that are sufficiently stringent to allow such hybridization, but without significant hybridization of noncomplementary nucleic acids. Such probes can be artificial, synthetic, or naturally occurring oligonucleotides, and typically contain naturally occurring nucleotides, but may contain modified or non-naturally occurring nucleotides such as those having universal bases and isobases. Two particularly useful isobases in probe nucleotides are 2'-deoxy-5-methylisocytidine (iC) and 2'-deoxy-isoguanosine (iG) (see U.S. Patent No. 6,001,983; No. 6,037,120; No. 6,617,106; and No. 6,977,161). In another embodiment, the probe can contain a nucleotide containing a removable base (such as uracil or 8-oxoguanine) so that treatment by uracil-DNA glycosylase (UDG) or formamidopyrimidine-DNA glycosylase (FPG), can lead to cleavage and degradation of unwanted or excess probes. Probes may permit a limited number of mismatched or degenerate positions as long as they are capable of hybridization for the purposes of the invention. Probes useful in the invention vary in length, according to the application, desired selectivity, and stringency used, and can be 5, 10, 15, 20, 25, 30, 35, or 40, 50, 60, 70, 80, 90, or 100 or more nucleotides.

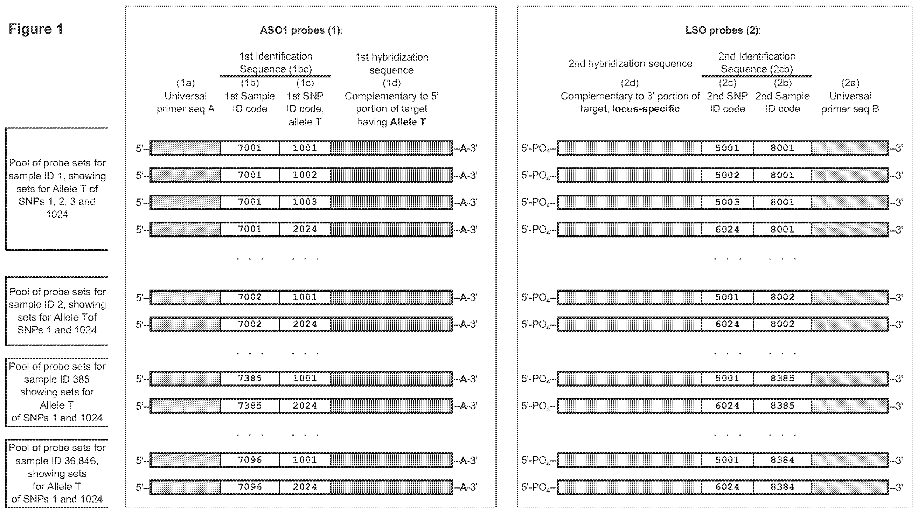

The probes have a "hybridization sequence" that is complementary or substantially complementary to a portion of the sequence of interest. Thus, in **Fig. 1****,** hybridization sequences are shown as **1d, 2d, and 3d** with the lines indicating their corresponding portions of the sequence of interest **(1d', 2d' 3d').** The hybridization sequence can be any length suitable for hybridization, and can be from about 5, 7, 10, 12, 15, 17, 20, 22, 25 nucleotides to about 10, 12, 14, 16, 18, 20, 22, 24, 30, 40, 50, 60, 80, 100, 200 or more nucleotides.

The hybridization sequences of probes **1** and **3** should correspond to different alleles at the sequence of interest, thus they can be termed "allele-specific oligonucleotides" or "ASOs". As discussed above, however, the "allele" can be any sequence of interest, for example a sequence characteristic for a species or genus of interest. It is desirable, but not necessary, that the ASO1 and ASO2 be able to discriminate between the two different alleles (or species) for the same sequence of interest, for example by having imperfect base-pairing at one or more nucleotide positions, such as a terminal base. If some cross-hybridization occurs, it can be useful to add one or more steps that provide additional layers of specificity, such as enzymatic ligation and/or extension, or selective termination, as discussed below. The hybridization sequences of probe **2** can be designed to be relatively nondiscriminating toward different alleles, and is thus sometimes termed a "locus-specific oligonucleotide" or "LSO" by convention.

An ordinarily skilled person would understand that with such an assay design, it is also possible to have additional LSOs that have sequences providing additional discrimination between different alleles or species. For example, while an ASO1 is paired with an LSO, the ASO2 may be paired with LSO2, and so on. Depending on the particular method desired, additional ASOs can also be provided, such as an ASO3 for allele A and ASO4 for allele G, or more for more complex polymorphisms or mixtures of species. For ease of discussion, however, the examples are shown with typical combination probes **1, 2, 3.**

The present application also relates to probe sets. An example of a "probe set" is an ASO1 **(1)** and an LSO **(2),** and optionally an ASO2 **(3)** or an LSO2. A probe set is typically provided for each combination of {samples} x {sequences of interest}. For example, an experiment to detect 6 SNPs in 8 samples can use 48 probe sets, each with 3 probes, for a total of 144 probes provided. Thus, the 48 probe sets can be described as a "pool of probe sets". Depending on the design of the experiment, however, not all 144 probes may be necessary to perform the method. The application relates to pools of probe sets, which may contain more than 100, more than 500, more than 1000 more than 5000, or more than 10,000 probe sets. Thus, the invention provides a high degree of flexibility for one of skill in the art to change the number of sequences of interest and samples to be assayed.

The probes can be labeled with a variety of detectable labels or primary labels, as is well understood in the art. It is also well understood that probes can be immobilized to a solid substrate to facilitate manipulation, as described below. One or more probes of a probe set may also be phosphorylated or otherwise modified to facilitate an enzymatic step, such as a joining step. Thus, for example, LSO probes are illustrated in phosphorylated form in the figures to allow convenient ligation in a later step.

### Identification sequences

In another embodiment, the probes described herein each have an "identification sequence" or "ID Seq". The ID Seq of the first probe is shown as **1bc** (or sometimes **1cb,** depending on the relative orientation); the ID Seq of the second probe is **2bc** or **2cb.** The ID Seq contains one or more separate nucleotide subsequences (represented as 4-digit numbers in the figures, e.g. 7001, 1001, 5001, 8001), sometimes referred to herein as a "codes", that are capable of identifying a particular sample (e.g. **1b, 2b),** a particular sequence of interest (e.g. **1c, 2c),** and/or distinguishing between particular alleles (e.g. **1c** vs. **3c).** In some embodiments, the ID Seq can contain between about 8 to about 11 nucleotides, or between 6 and 13, 10 and 100, or 30 and 50 nucleotides.

In one aspect, the identification sequence contains a sample code (e.g. **1b, 2b),** and a separate sequence-of-interest code (e.g. **1c, 2c).** In **Fig. 1****,** for example, the ASO1 probe has an identification sequence encompassing the two subsequences: "Sample ID code" **(1b)** and a SNP/allele code **(1c).** Similarly, the identification sequence of the LSO probe in **Fig. 1** is shown by a LSO-SNP code and "ID code". Representative lengths for the Sample ID codes and SNP/Allele codes can be 4, 5, or 6 nucleotides to 5 to 6 nucleotides.

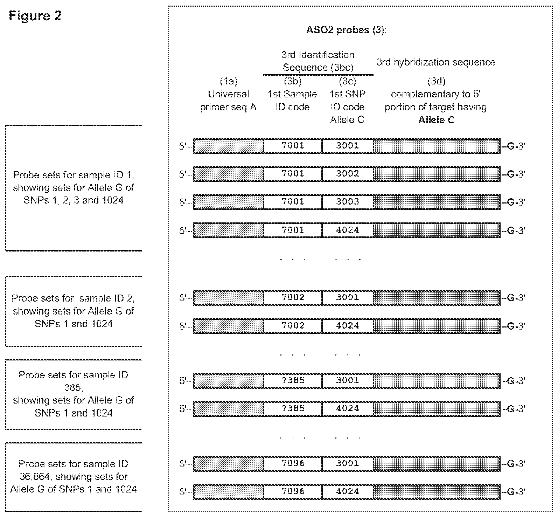

**Figs. 2** to **4** also illustrate probes with identification sequences, where the nucleotide sequences are represented by unique 4-digit numbers, such as 7 0 0 1, 7002, 7385 5 or 7096 for various "1st Sample ID codes" **(1b),** and 1001... 2024 for various "1st SNP ID codes" **(1c).** Thus in these figures, **(1b)** and **(1c)** combined are the ID Seq **(1b-1c)** for the ASO1. Similarly, the identification sequence is **2c-2b** for the LSO and **3b-3c** for the ASO2.

In another aspect, the identification sequence can be a single, undivided sequence, where two or more pieces of information are embedded within a single sequence. For example, the sequence "123456" can be used to identify sample "135" and sequence "2 4 6", or can be used with an algorithm and pre-defined parameters to identify sample 972 (integer multiple of 127) and sequence 12 (modulus).

In another aspect, the hybridization sequence itself can serve as the identification sequence, or alternatively the hybridization sequence and the identification sequence can overlap. Any spatial arrangement of the hybridization sequence and the identification sequence versus each other is contemplated in the invention.

Accordingly, the application relates to an ASO1 "first probe" having (in one embodiment, from 5' to 3') a first identification sequence **(1bc)** and a first hybridization sequence **(1d)** complementary to a first portion of a sequence of interest (**1d'**). The application also relates to an LSO "second probe" having (in one embodiment, from 5' to 3'), a second hybridization sequence **(2d)** complementary to a second portion of a sequence of interest **(2d')** and a second identification sequence **(2cb).**

### Contacting samples with probes

Having the sample and the probe sets described above, the method provides for contacting each sample with a plurality of probe sets, as illustrated in **Figs. 3a** and **3b****.** As used herein, the term "contacting" refers to exposing the probes to the sample under conditions that permit the probes to hybridize to the nucleic acids in the sample if they are sufficiently complementary.

Conditions for hybridization in the present invention are generally high stringency conditions as known in the art, although different stringency conditions can be used. Stringency conditions have been described, for example, in Sambrook et al., *Molecular Cloning: A Laboratory Manual,* 3d ed. (2001) or in Ausubel et al., *Current Protocols in Molecular Biology* (1998). High stringency conditions favor increased fidelity in hybridization, whereas reduced stringency permit lower fidelity. Stringent conditions are sequence-dependent and will be different in different circumstances. Longer sequences hybridize specifically at higher temperatures. An extensive guide to the hybridization of nucleic acids is found in Tijssen, "Overview of principles of hybridization and the strategy of nucleic acid assays" in *Techniques in Biochemistry and Molecular Biology-Hybridization with Nucleic Acid Probes* (1993). Generally, stringent conditions are selected to be about 5-10 C° lower than the thermal melting point (Tₘ) for the specific sequence at a defined ionic strength and pH. The Tₘ is the temperature (under defined ionic strength, pH and nucleic acid concentration) at which 50% of the probes complementary to the target hybridize to the target sequence at equilibrium (i.e., as the target sequences are present in excess, at Tₘ, 50% of the probes are occupied at equilibrium). Examples of stringent conditions are those in which the salt concentration is less than about 1.0 M sodium ion, typically about 0.01 to 1.0 M sodium ion concentration (or other salts) at pH 7.0 to 8.3 and the temperature at least about 30 °C for short probes (e.g. 10 to 50 nucleotides) and at least about 60 °C for long probes (e.g. greater than 50 nucleotides). Stringent conditions may also be achieved with the addition of helix-destabilizing agents such as formamide. Stringency can be controlled by altering a step parameter that is a thermodynamic variable such as temperature or concentrations of formamide, salt, chaotropic salt, pH, and/or organic solvent. These parameters may also be used to control non-specific binding, as is generally outlined in U.S. Patent No. 5,681,697. Thus it may be desirable to perform certain steps at higher stringency conditions to reduce non-specific binding.

The contacting step can be performed in a solution-phase process in the absence of solid supports. Alternatively, the contacting step can be performed with immobilized sample nucleic acids or with immobilized probes.

### Hybridization complexes

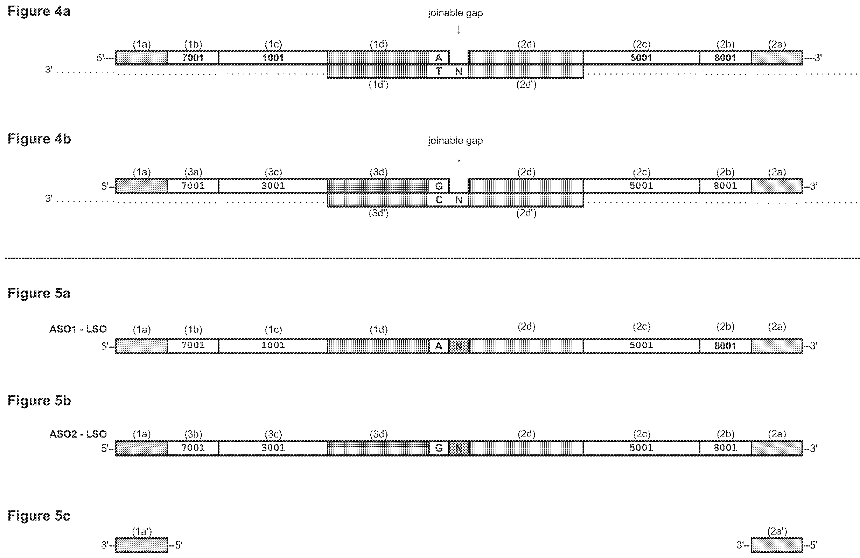

After the sample has been contacted with the probe sets under conditions that allow hybridization, various hybridization complexes (as in **Fig. 4a** and **4b****)** may be formed. In one aspect, the first probe is allowed to hybridize (or not hybridize) to the sample nucleic acid. In another aspect, hybridization occurs between the sample nucleic acids and two probes from a probe set. In one embodiment, the hybridization properties are similar between the first and second (and optional third) hybridization sequences to their respective first and second (and optional third) portions of the sequence of interest to allow them to hybridize under the same or compatible conditions and/or in the same reaction.

As exemplified in **Fig. 4a****,** an ASO1 **(1)** may hybridize to a sequence of interest when the ASO1 contains a first hybridization sequence **(1d)** that is sufficiently complementary to a first portion **(1d')** of the sequence of interest. Similarly, an LSO **(2)** may hybridize to the same sequence of interest when the LSO contains a second hybridization sequence **(2d)** that is sufficiently complementary to a second portion **(2d')** of the sequence of interest. Thus, a hybridization complex will be formed by the ASO1 and the LSO to the sequence of interest **(1d'-2d').** In some embodiments, the ASO1 and LSO may hybridize adjacently or one or more nucleotides away from each other. In either case, the gap in the sugar-phosphate backbone can be described as a "joinable gap".

In other cases, the LSO may hybridize, but the hybridization sequence **(3d)** of ASO2 instead will hybridize to the first portion **(3d')** as in **Fig. 4b****.** In this case, the hybridization complex will be formed by the ASO2 and the LSO to the sequence of interest **(3d'-2d'),** which may or may not have a joinable gap.

Upon formation of such hybridization complexes, an optional washing step may be performed to remove unhybridized probes and sample nucleic acids.

### Joining probes

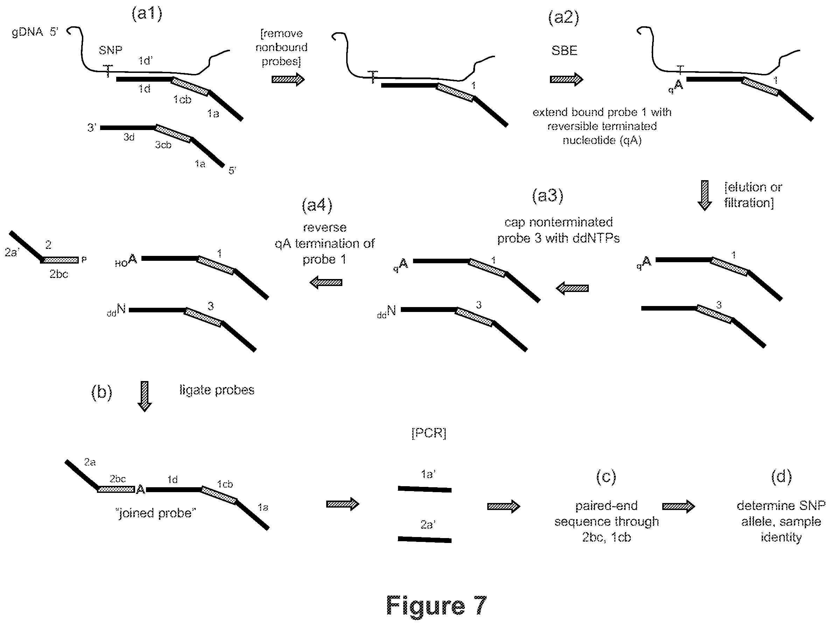

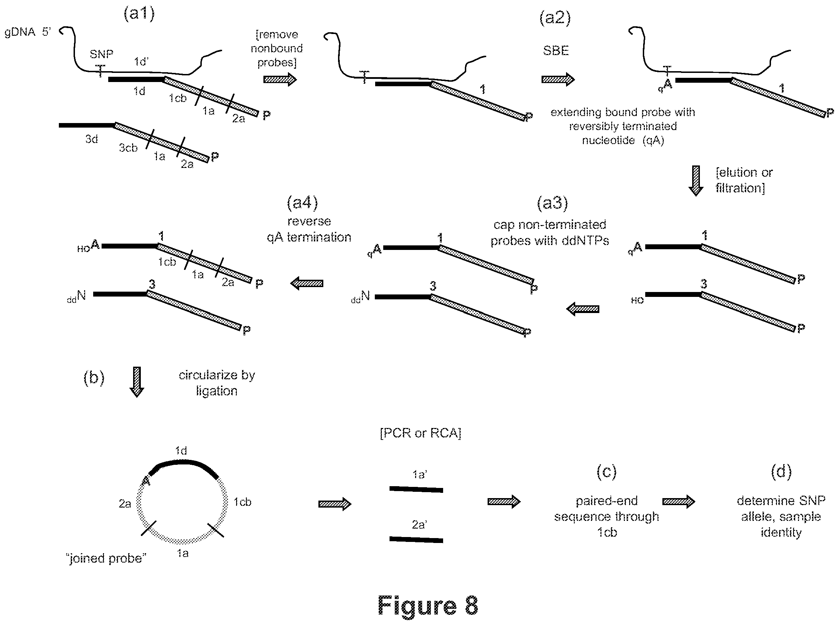

The two probes hybridized to the sequence of interest are then joined to form a "joined probe". The term "joining" means performing any combination of steps that result in a single, covalently joined molecule. In one aspect, joined probes ASO1-LSO and ASO2-LSO are illustrated in **Fig. 5****.** In another aspect, a joined probe **2a-2bc-A-1d-1cb-1a** is illustrated in **Fig. 7****.** In yet another aspect, a joined probe is illustrated in **Fig. 8****,** where the probe is self-ligated head-to-tail to form a single, covalently joined molecule having **1d-2a-1a-1cb.** The joining can be performed by a variety of techniques, such as chemical ligation or cross-linking, or by enzymatic techniques involving ligation, polymerase extension, endonuclease reverse-reaction or any combination of these. When the two probes are separated by one or more nucleotides, the invention makes use of intermediate oligonucleotides or linker molecules to allow joining of the two probes

When the joining step is performed enzymatically, it can provide an added degree of specificity to the method, in addition to the specificity obtained from the hybridization step. Many ligases and polymerases, for example, will not catalyze a joining step unless the nucleotides contacting or near the active site are perfectly base-paired.

### - by ligation

In one embodiment, a ligase can be provided to form a covalent bond between the two probes when they are hybridized adjacently to the same strand. Joining of adjacent probes may be achieved by chemical or enzymatic means, such as by a double-strand ligase (like T4 DNA ligase) or a single-strand ligase, depending on the configuration of the probes. Examples of single-strand ligases include CircLigase^{™} (Epicentre), and T4 RNA ligase 1, depending on the configuration of probes.

Joining of two probes which are not hybridized adjacently to the same strand may be done by contacting the probes with a single strand polynucleotide ligase. In one aspect, the joining of two probes may be inhibited by the addition oaf a terminator base (such as a dideoxynucleotide) to one of the probes, thereby preventing subsequent extension and/or ligation from occurring. In some embodiments of the invention, a reversible or ligatable terminator base is added to a probe that is hybridized adjacently to a SNP so that the addition of the reversible or ligatable terminator base complements the allele nucleotide present at the SNP. Joining may also involve the addition of nucleotides or other chemical moieties that were not present in the first and second probes when they were separate molecules. Alternatively, the second probe may serve as primer for AS-PCR, also known as amplification refractory mutation system (ARMS).

In some embodiments, probes are designed to allow the use of OLA (or rolling circle amplification (RCA) as generally described in Baner et al. (1998) Nuc. Acids Res. 26:5073-5078; Barany, F. (1991) Proc. Natl. Acad. Sci. USA 88:189-193; and Lizardi et al. (1998) Nat. Genet. 19:225-232, This finds particular use in genotyping reactions, for the identification of nucleotides at a SNP position, for example. The basic OLA method can be run at least two different ways: in a first embodiment, only one strand of a double-stranded sample nucleotide sequence is used as a template for ligation; alternatively, both strands may be used-the latter is generally referred to as Ligation Chain Reaction or LCR. The discussion below focuses on OLA, but as those skilled in the art will appreciate, this can easily be applied to LCR as well.

The first probe is hybridized to the first portion of the sequence of interest and a second probe is hybridized to the second portion. If the first ligation probe has a base perfectly complementary to its position on the sequence of interest, and the adjacent base on the second probe has perfect complementarity to its adjacent position on the sequence of interest, a ligation structure is formed so that the two probes can be ligated together to form a joined probe. If this complementarity does not exist, no ligation structure is formed and the probes are not ligated together to an appreciable degree. This may be done using heat cycling, to allow the ligated probe to be denatured off the sequence of interest so that it may serve as a template for further reactions. In addition, this method may be performed using three ligation probes or ligation probes that are separated by one or more nucleotides, if dNTPs and a polymerase are added (this is sometimes referred to as "Genetic Bit" analysis).

In some embodiments, LCR is performed for two strands of a double-stranded target sequence. The target sequence is denatured, and two sets of probes are added: one set as outlined above for one strand of the sequence of interest, and a separate set (i.e. third and fourth probes) for the other strand of the sequence of interest. In this embodiment, the method uses each set of probes with a different adapter; this can serve as an additional specificity control-a "positive" called only if both strands are detected.

### - with extension

In another embodiment, the two probes are separated by one or more nucleotides while hybridized to the sequence of interest. The gap can be a single nucleotide or can be a gap of more than one nucleotide. The extension can be carried out at the 3' end of the first probe when hybridized to a sample nucleic acid. The sample nucleic acid acts as a template directing the type of modification, for example, by base-pairing interactions that occur during polymerase-based extension of the first probe to incorporate one or more nucleotides. Such a scenario can be used, for example, in detecting the presence of a SNP. Extensions can be carried out to modify first probes that have free 3' ends, for example, when bound to the sample nucleic acid. Exemplary approaches that can be used include, without limitation, allele-specific primer extension (ASPE) and single base extension (SBE). A nucleic acid, nucleotide or nucleoside having a reversible blocking group on a 2', 3' or 4' hydroxyl, a peptide linked label or a combination thereof can be used in such methods. For example the nucleic acid, nucleotide or nucleoside can be included in the first probe. Additionally or alternatively, the nucleic acid, nucleotide or nucleoside can be used to modify the free 3' ends in the extension reactions.

### - with single-base extension (SBE)

In a further embodiment a single base extension (SBE) can be used in conjunction with an oligonucleotide ligation assay (OLA) to produce the joined probe. Briefly, SBE utilizes a first probe that hybridizes to the sequence of interest, adjacent or within a few nucleotides of the polymorphism of interest. A polymerase is used to extend the 3' end of the probe. Based on the fidelity of the enzyme, a nucleotide is only incorporated into the first probe if it is complementary to the sequence of interest. SBE can be carried out under known conditions such as those described in U.S. Patent Application No. 09/425,633. As will be appreciated by those skilled in the art, the configuration of an SBE reaction can take on any of several forms. For example, SBE can be performed on a surface or in solution, wherein the newly synthesized strands can be amplified in a subsequent step.

If desired, the nucleotide can be derivatized so that no further extensions can occur. Alternatively, the nucleotide can be derivatized using a blocking group (including reversible blocking groups) so that only a single nucleotide is added. A nucleotide analog useful for SBE can include a dideoxynucleoside-triphosphate (also called deoxynucleotides or ddNTPs, i.e. ddATP, ddTTP, ddCTP and ddGTP), or other nucleotide analogs that are derivatized to be chain-terminating. For example, nucleotides containing cleavable peptide linkers linking a dye and/or blocking groups (removable or not) can be used for SBE. Exemplary analogs are dideoxy-triphosphate nucleotides (ddNTPs) or acyclo terminators. Generally, a set of nucleotides comprising ddATP, ddCTP, ddGTP and ddTTP can be used. As will be appreciated by those skilled in the art, any number of nucleotides or analogs thereof can be added to a primer, as long as a polymerase enzyme is able to incorporate a particular nucleotide.

A nucleotide used in an SBE method can further include a detectable label, such as the ones particularly described herein. The labels can be attached via a variety of linkages, such as a peptide linkage. If a primary label is used, the use of secondary labels can also facilitate the removal of unextended probes in particular embodiments.

When SBE is performed, the invention makes use of an extension enzyme, such as a DNA polymerase. Suitable DNA polymerases include Klenow fragment of DNA polymerase I, Sequenase™ 1.0 and Sequenase™ 2.0 (U.S. Biochemical), T5 DNA polymerase, Phi29 DNA polymerase, and Thermosequenase™ (Taqwith the Tabor-Richardson mutation). Modified versions of these polymerases that have improved ability to incorporate a nucleotide analog may be used if so desired. If the nucleotide is complementary to the base of the detection position of the target sequence, which is adjacent to the extension primer, the extension enzyme will add it to the extension primer. Thus, the extension primer is modified, i.e. extended, to form a modified primer.

### - using reversible terminators

In another aspect, the SBE reaction can be used with reversible terminators to obtain the joined probe. As illustrated in **Fig. 7****,** the sample is contacted with a plurality of probe sets, each probe set comprising at least a first probe having a first identification sequence **(1cb)** and a first hybridization sequence **(1d)** complementary to a sequence of interest (**1d'**). As illustrated, the sequence of interest is the T allele of a SNP. At this stage, there may be many other bound or unbound probes directed to other alleles. Optionally, the annealed complex can be washed or filtered to remove unhybridized probes. Accordingly, a number of different techniques may be used to facilitate the removal of unextended probes, including methods based on removal of unreacted probes by binding to a solid support, protecting the reacted probes and degrading the unextended ones, and separating the unreacted from the reacted probes, for example by using a molecular-weight cut-off (MWCO) filter plate.

Step (a2) shows a discriminating SBE-type extension reaction where the hybridized first probe **(1)** is extended by incorporating a reversibly terminated base such as qA, where q stands for the termination moiety, and A stands for the nucleotide complementary to the SNP. When other alleles are to be detected, other reversibly terminated complementary bases such as qT, qC, qG, or terminated isobases q-isoC or q-isoT can be used. The result is a reversibly terminated first probe. Probes **(3)** that are not specific to the sequence of interest **(1d')** are not hybridized and not extended, and will therefore lack a terminator. Optionally, the terminated probes can be eluted or otherwise separated from the gDNA. In one embodiment, this can be accomplished by using a biotinylated terminator, which facilitates manipulation and washing steps to remove non-terminated probes and reduce nonspecific background signal.

In step (a3), any remaining probes **(3)** that are not specific to the sequence of interest can be capped to prevent subsequent ligation. This can be accomplished by adding ddNTPs using terminal dideoxynucleotidyl transferase (TdT). Optionally, the ddNTPs can be biotin-labeled to facilitate removal.

As illustrated in step (a4), the termination of probes **(1)** is reversed, so that the probe is available for ligation to ligatable terminus, for example a phosphorylated second probe **(2).** For example, a qA terminator can be removed, leaving a 3'-OH. Another example is a 3'-amino group that can be chemically ligated to a phosphorylated second probe **(2),** where the 5' phosphate group of the second probe can be activated with a carbodiimide and imidazole or alternatively cyanogen bromide. Ligation during step (b) then produces the joined probe, which contains identification sequences **1cb** and **2bc.**

In another embodiment, illustrated in **Fig. 8****,** the first probe **(1)** contains the hybridization sequence **(1d),** an identification sequence **(1bc)** and further has universal primer sequences **1a** and **2a,** discussed further below. Upon ligation, a circularized molecule is formed that serves as the "joined probe". In this sense, the probe is considered joined because the first probe has been ligated to another ligatable terminus on the same (or another) first probe. Circularization can be accomplished with ssDNA ligase. Joined probes in circularized form can be advantageous during subsequent amplification steps using PCR or rolling circle amplification (RCA). The circular probes may optionally be linearized for decoding, as discussed below.

### - with GoldenGate

A particularly useful method for joining is the GoldenGate^{®} assay chemistry, which combines a polymerase extension step and a ligation step. This chemistry and other chemistries for joining are described in U.S. Patent No. 7,582,420 with a methylation-detection variant in Patent No. 7,611,869.

After joining the probes, it may be useful to purify the joined probes through a combination of denaturing and washing steps. Optionally, the joined probes can be amplified to facilitate detection by a variety of methods.

### Optional Amplification

In one embodiment, the application relates to compositions and methods for amplification. Suitable amplification methods include both target amplification and signal amplification. Target amplification involves the amplification (i.e. replication) of the target sequence to be detected, resulting in a significant increase in the number of target molecules. Target amplification strategies include but are not limited to the polymerase chain reaction (PCR) as generally described herein, strand displacement amplification (SDA) as generally described in Walker et al., in Molecular Methods for Virus Detection, Academic Press, Inc., 1995, and U.S. Patent No. 5,455,166 and No. 5,130,238, and nucleic acid sequence based amplification (NASBA) as generally described in U.S. Patent No. 5,409,818; Sooknanan et al., Nucleic Acid Sequence-Based Amplification, Ch. 12 (pp. 261-285) of Molecular Methods for Virus Detection, Academic Press, 1995; and *"*Profiting from Gene-based Diagnostics", CTB International Publishing Inc., N.J., 1996.

Alternatively, rather than amplify the target, alternate techniques use the target as a template to replicate a signaling probe, allowing a small number of target molecules to result in a large number of signaling probes, that then can be detected. Signal amplification strategies include the ligase chain reaction (LCR), cycling probe technology (CPT), invasive cleavage techniques such as Invader™ technology, Q-Beta replicase (QβR) technology, and the use of "amplification probes" such as "branched DNA" that result in multiple label probes binding to a single target sequence.

All of these methods require a primer nucleic acid (including nucleic acid analogs) that is hybridized to a target sequence to form a hybridization complex, and an enzyme is added that in some way modifies the primer to form a modified primer. For example, PCR generally requires two primers, dNTPs and a DNA polymerase; LCR requires two primers that adjacently hybridize to the target sequence and a ligase; CPT requires one cleavable primer and a cleaving enzyme; invasive cleavage requires two primers and a cleavage enzyme; etc.

### - with universal primer sequences

To facilitate amplification, the probes themselves can have an optional primer sequence, exemplified by **(1a)** or (**2a**) in the figures, which can allow primers **(1a', 2a'),** such as PCR primers, to hybridize to the sequences. The selection of primer sequences can vary between different probes, but will be determined by the particular design of the amplification step. In a preferred embodiment, the priming sites are preferably located at the 5' and 3' termini of the joined probe, as shown in the figures so that sequences flanked by priming sequences will be amplified.

A primer sequence can be described as "universal" when the same primer sequence appears among a plurality or even all of a type of probe (e.g. ASO1s, LSOs, ASO2s), so that a small set of primers can be used for amplification many or all of the joined probes in the same reaction. The universal priming sequence can be between 15 and 25 nucleotides in length in some embodiments, and between 17 and 20 nucleotides in other embodiments. In one embodiment, a single primer sequence **(1a)** is the same for all ASOs used in an experiment, as shown in the figures. However, it can be useful for ASO1s and ASO2s to have different primer sequences for use with different primers, for example when the primers are labeled differently.

In general, a target nucleic acid is added to a reaction mixture that comprises the necessary amplification components, and a modified primer is formed. The modified primer can comprise a detectable label, such as a fluorescent label, which is either incorporated by the enzyme or present on the original primer. As required, the unreacted primers are removed, in a variety of ways, as will be appreciated by those skilled in the art and outlined herein. The hybridization complex is then disassociated, and the modified primer is detected and optionally quantitated by a microsphere array. In some cases, the newly modified primer serves as a target sequence for a secondary reaction, which then produces a number of amplified strands, which can be detected as outlined herein.

Accordingly, the reaction starts with the addition of a primer nucleic acid to the target sequence which forms a hybridization complex. Once the hybridization complex between the primer and the target sequence has been formed, an enzyme, sometimes termed an "amplification enzyme", is used to modify the primer. As for all the methods outlined herein, the enzymes may be added at any point during the assay, either prior to, during, or after the addition of the primers. The identity of the enzyme will depend on the amplification technique used, as is more fully outlined below. Similarly, the modification will depend on the amplification technique, as outlined below.

Once the enzyme has modified the primer to form a modified primer, the hybridization complex is disassociated. In one aspect, dissociation is by modification of the assay conditions. In another aspect, the modified primer no longer hybridizes to the target nucleic acid and dissociates. Either one or both of these aspects can be employed in signal and target amplification reactions as described below. Generally, the amplification steps are repeated for a period of time to allow a number of cycles, depending on the number of copies of the original target sequence and the sensitivity of detection, with cycles ranging from 1 to thousands, with from 10 to 100 cycles being preferred and from 20 to 50 cycles being especially preferred. When linear strand displacement amplification is used cycle numbers can reach thousands to millions.

After a suitable time of amplification, unreacted primers are removed, in a variety of ways, as will be appreciated by those skilled in the art and described below, and the hybridization complex is disassociated. In general, the modified primer comprises a detectable label, such as a fluorescent label, which is either incorporated by the enzyme or present on the original primer, and the modified primer is detected by any of the methods as known to the skilled artisan and include but are not limited to the methods described herein

### - PCR

In one embodiment, the target amplification technique is PCR The polymerase chain reaction (PCR) is widely used and described, and involves the use of primer extension combined with thermal cycling to amplify a target sequence; see U.S. Patent No. 4,683,195 and No. 4,683,202, and PCR Essential Data, C.R. Newton, ed. (J.W. Wiley & Sons 1995).

In addition, there are a number of variations of PCR which also find use in the invention, including quantitative competitive PCR (QC-PCR), arbitrarily primed PCR (AP-PCR), immuno-PCR, Alu-PCR, PCR single-strand conformational polymorphism" (PCR-SSCP), reverse transcriptase PCR (RT-PCR), biotin capture PCR, vectorette PCR, panhandle PCR, PCR select cDNA subtraction, and allele-specific PCR, among other PCR variations known in the art. In some embodiments, however, PCR is not preferred for amplification, and other amplification methods can be used, such as isothermal methods or methods that do not rely on thermal cycling.

Accordingly, the PCR reaction requires at least one PCR primer, a polymerase, and a set of dNTPs. As outlined herein, the primers may comprise the label, or one or more of the dNTPs may comprise a label.

In one embodiment asymmetric PCR is performed. In this embodiment, unequal concentrations of primers are included in the amplification reaction. The concentrations are designed such that one primer is in excess or is saturating, while the other primer is limiting or is at a sub-saturating concentration.

In one embodiment, PCR primers for amplification of a plurality of target nucleic acids are immobilized on a single bead. That is, at least one of the first and second PCR. primer pairs is immobilized to a bead or microsphere. The microsphere is contacted with a sample and PCR performed as described herein. Detection of the amplified product or products is accomplished by any of the detection methods described herein, but in a preferred embodiment, detection proceeds by hybridization with allele specific oligonucleotides. That is, upon amplification of the target nucleotides, the immobilized PCR product is hybridized with oligonucleotides that are complementary to the amplified product.

In one embodiment the allele-specific oligonucleotides contain distinguishable labels. As a result of hybridization between the allele specific oligonucleotides and the amplified product(s), detection of a particular label provides an indication of the presence of a particular target nucleic acid in the sample.

In one embodiment, the PCR primers are designed to amplify different genomic markers. That is, markers such as translocations or other chromosomal abnormalities are targeted for amplification. In an additional embodiment, the primers are designed to amplify genomic regions containing SNPs. As such, the resulting hybridization with allele specific oligonucleotides provides an indication of the marker or SNP. In one embodiment, a plurality of markers or SNPs is detected on each bead. That is, at least two markers or SNPs are detected on each bead.

In general, as is more fully outlined herein, the capture probes or oligonucleotides on the beads of the array are designed to be substantially complementary to the extended part of the primer; that is, unextended primers will not bind to the capture probes. Alternatively, as further described herein, unreacted probes may be removed prior to addition to the array.

In one embodiment the amplification reaction is a multiplex amplification reaction as described herein. In one embodiment the amplification reaction uses a plurality of PCR primers to amplify a plurality of target sequences. In this embodiment, the plurality of target sequences are simultaneously amplified with the plurality of amplification primer pairs.

In an alternative embodiment, the multiplex PCR reaction uses universal primers as described herein. That is, universal PCR primers hybridized to universal priming sites on the target sequence and thereby amplify a plurality of target sequences. This embodiment is potentially preferred because it requires only a limited number of PCR primers. That is, as few as one primer pairs can amplify a plurality of target sequences.

In one embodiment, a multiplex amplification reaction such a "bridge amplification" is used to amplify the target sequences, i.e. joined probes, as described in WO 98144151, WO 96/04404, WO 07/010251, and U.S. Patent No. 5,641,658, No. 6,060,288, No. 6,090,592, No. 6,468,751, No. 6,300,070, and No. 7,115,400,

Bridge amplification localizes the target and one or more primers within sufficient proximity so that complementary sequences hybridize. Following hybridization, the single stranded regions are extended with, for example, a template directed nucleic acid polymerase to modify each molecule to include the sequence of the extension product. Multiple rounds of this extension procedure will result in the synthesis of a population of amplicons. Because the target nucleic acid and the probe or primer is immobilized at a feature and its adjacent surrounding area, the amplicons become highly localized and concentrated at the area of the discrete feature.

### Decoding ID sequences

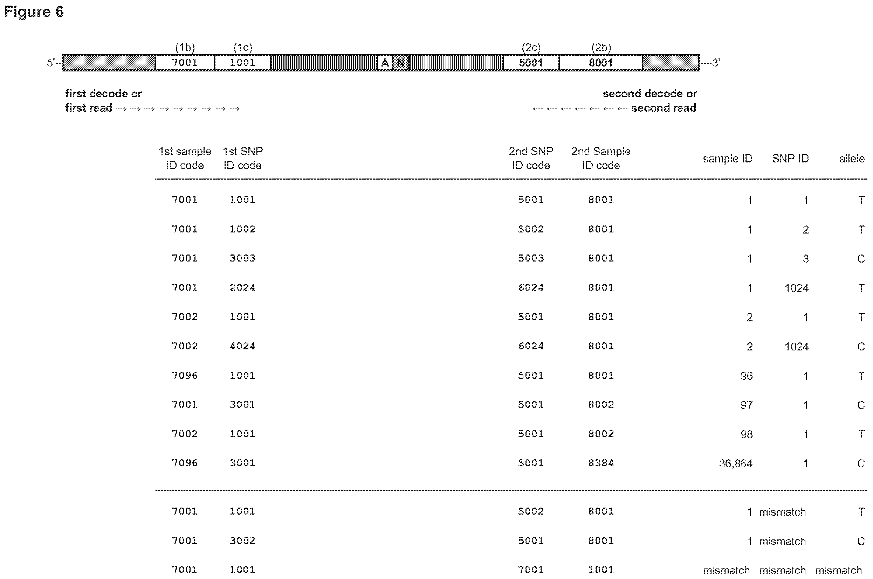

Whether or not the joined probes are amplified, a joined probe will be formed only if the two probes hybridized correctly to the sequence of interest, and they are correctly base-paired to allow the polymerase and/or ligase to perform an enzymatic joining step. Thus, while there may be undesirable hybridization complexes that are formed, the physical existence of a joined probe is an indication that the corresponding sequence of interest was present in the sample nucleic acids. Accordingly, the invention provides for determining the identification sequences **(1b-1c** and **2c-2b)** that have been brought together to form a joined probe (ASO1-LSO). Determining the identity of the first and second sample identification sequences can be performed by standard methods available in the art and described herein below. As exemplified in **Fig. 6****,** the joined probe (or amplification product thereof) has a 5' set of codes **(1b-1c)** and a 3' set of codes **(2c-2b).** In **Fig. 7****,** the codes **2bc** and **1cb** can be decoded; in **Fig. 8****,** codes **1cb.** These codes can be decoded by a variety of methods. In the methods of the invention, decoding the identification sequences for a plurality of joined probes comprises paired-end sequencing of the first and second identification sequences.

The term "decoding" means performing any combination of steps for ascertaining the nucleotide composition of a sequence, such as the identification sequence and/or hybridization sequence of joined probes described herein and correlating that sequence to a sample, a sequence of interest, a specific allele, and/or any other identifying information embedded within the "code" of the sequence.

The "code" of the sequence refers the contiguous series of nucleotides making up the nucleic acid sequence. The "code" can represent the sequence of interest the probe set was assaying for and/or the sample which has the sequence of interest. Steps for ascertaining the nucleotide composition of a sequence include any method that results in identifying the sequence of nucleotides at a given location within a nucleic acid, such as a joined probe.

### - by decoding probes

In one aspect , ascertaining the nucleotide composition of a nucleic acid includes hybridizing a decoding oligonucleotide to the identification sequence, wherein the hybridization of the decoding oligonucleotide is detected and that detection is an indication of the sequence composition of the of the nucleic acid. In one embodiment, the specificity for sequence analysis is provided by a cleavage enzyme. There are a variety of enzymes known to cleave at specific sites, either based on sequence specificity, such as restriction endonucleases, or using structural specificity, such as is done through the use of invasive cleavage technology.

In one embodiment, enzymes that rely on sequence specificity are used. In general, these systems rely on the cleavage of double stranded sequence containing a specific sequence recognized by a nuclease, preferably an endonuclease including resolvases. These systems may work in a variety of ways. In one embodiment, a labeled readout probe (generally attached to a bead of the array) is used; the binding of the target sequence forms a double stranded sequence that a restriction endonuclease can then recognize and cleave, if the correct sequence is present. The cleavage results in the loss of the label, and thus a loss of signal.

### - by paired-end sequencing

It may be observed that in joined probes, exemplified in **Fig. 6** and **Fig. 7****,** there are a 5' set of codes **(1b-1c)** and a 3' set of codes **(2c-2b).** These codes can be decoded by various sequencing methods, such as sequencing all or a part of the joined probe. To facilitate sequencing methods, the probes can be designed to incorporate sequences that are used with sequencing primers in various sequencing methods. These sequences can be used in various positions on a probe, but as an example, sequencing-primer sequences can be present in probe **1** between **1a** and **1cb,** and in probe **2** between **2bc** and **2a.** In the reversible terminator embodiment of **Fig. 8****,** the sequencing-primer sequences can be present in probe 1 between **1d** and **1a,** and at the 3' end of **2a**.

In the invention, sequencing of the nucleic acid or more particularly paired-end sequencing of the nucleic acid, can be used to ascertain the nucleotide composition of a nucleic acid. Methods for sequencing nucleic acids, particularly paired-end sequencing, are well known to those skilled in the art. Methods for conducting such paired-end sequencing are described in U.S. Pub. 2007/0015200, U.S. Pub. 2009/0181370, WO07091077. WO08041002 and U.S. Patent No. 7,601,499,

Other methods, some of which are adapted to paired-end sequencing include, without limitation, sequencing by synthesis (SBS), including pyrosequencing, sequencing by ligation, sequencing by hybridization, chain terminating Sanger sequencing, and the like.

As paired-end sequencing is possible on high-throughput sequencing instruments, an additional benefit of the invention is that the products of individual joining steps can be combined into a single mixture containing products from different samples, which can be sequenced in a single combined step, rather than performing individual sequencing steps for each joining step.

### Optional immobilization

In one embodiment, the sequence of interest, probe or primer, including a modified primer, is attached to a substrate or solid support. By "substrate" or "solid support" or other grammatical equivalents herein is meant any material that is appropriate for or can be modified to be appropriate for the attachment of the target sequences. As will be appreciated by those skilled in the art, the number of possible substrates is very large. Possible substrates include, but are not limited to, glass and modified or functionalized glass, plastics (including acrylics, polystyrene and copolymers of styrene and other materials, polypropylene, polyethylene, polybutylene, polyurethanes, Teflon^{™}, etc.), polysaccharides, nylon or nitrocellulose, ceramics, resins, silica or silica-based materials including silicon and modified silicon, carbon, metals, inorganic glasses, plastics, optical fiber bundles, and a variety of other polymers. Magnetic beads and high throughput microtiter plates are particularly preferred.

The composition and geometry of the solid support vary with its use. In this particular embodiment, supports comprising microspheres or beads are preferred for the first solid support. By "microspheres" or "beads" or "particles" or grammatical equivalents herein is meant small discrete particles. The composition of the beads will vary, depending on the class of bioactive agent and the method of synthesis. Suitable bead compositions include those used in peptide, nucleic acid and organic moiety synthesis, including, but not limited to, plastics, ceramics, glass, polystyrene, methylstyrene, acrylic polymers, paramagnetic materials, thoria sol, carbon graphite, titanium dioxide, latex or cross-linked dextrans such as Scpharosc^{™}, cellulose, nylon, cross-linked micelles and Teflon^{™}, as well as any other materials outlined herein for solid supports may all be used. *"Microsphere Detection Guide"* from Bangs Laboratories, Fishers IN is a helpful guide. Preferably, in this embodiment, when complexity reduction is performed, the microspheres are magnetic microspheres or beads.

The beads need not be spherical; irregular particles may be used. In addition, the beads may be porous, thus increasing the surface area of the bead available for assay. The bead sizes range from nanometers, i.e. 100 nm, to millimeters, i.e. 1 mm, with beads from about 0.2 micron to about 200 microns being preferred, and from about 0.5 to about 5 microsn being particularly preferred, although in some embodiments smaller beads may be used.

In some embodiments, the methods of the present invention can be used in conjunction with a flow cell. A "flow cell" is a solid phase support that has about eight or more lanes. Each lane can accommodate approximately six million clonally amplified clusters and is designed to present nucleic acids in a manner that facilitates access to enzymes while ensuring high stability of surface-bound templates and low non-specific binding of labeled nucleotides. Indeed, the commercial trend in sequencing instruments appears to be the use of flow cells because of the high throughput analysis that can be achieved with such systems.

The use of a flow cell in the methods presents a cost-effective method for assaying multiple samples for a plurality of sequences of interest. For example, if approximately 6 million clonally amplified clusters are available per lane in a flow cell, 96 SNPs are assayed per sample, and approximately a 15-nucleotide-sequence depth is performed, one sequencing lane in a flow cell can be used to measure 4166 samples. If the running cost of each lane is about $200 (assuming a read depth of approximately 10 bases from each end), then the sequencing readout will cost about 5 cents per sample (for 96 SNPs). Furthermore, assuming a sequencer can process a flow cell having 8 lanes in two days of processing, about 4000 samples x 8 lanes will result in 32,000 samples processed in two days.

In another example, using the probe combinations and methods described herein, to genotype 96 SNPs, (40 x 2 x 96) + (96 x 100) = 17,280 oligonucleotides can be designed and synthesized. Of these oligonucleotides, 9600 can be 5'-phos modified. The 17,280 oligonucleotides will be used to constitute 4000 different oligonucleotide pools, each having a unique sample ID and 96 SNP IDs. Thus, each pool can be used to assay 96 SNPs in one genomic DNA sample. In one embodiment, the 4000 oligonucleotide pools can be used to assay 4000 DNA samples simultaneously, then pooled together, and sequenced on a flow cell (one lane is enough to read the 4000 samples, see above).

### Determining

Having decoded the ID sequences of a joined probe (or its amplification products), the invention provides methods for extracting information from these codes to determine the presence of the sequence of interest and the identity of the sample. Here, the sequence of interest can be an allele of a polymorphism, a species-specific sequence, or any other sequence whose presence/absence or quantitative level is to be detected. For purposes of explanation, however, the allele aspect of the method will be exemplified. Although a great variety of coding/decoding schemes can be selected or designed to suit the goals and needs of a particular experiment, a representative scheme has been illustrated in the figures and are explicated as follows.

### - SNP / allele information

In this scheme, the 2nd SNP ID code (**2c**) on the LSO is used to indicate the SNP whose presence or absence is being detected. When a 1st SNP ID code **(1c)** is decoded on the same joined probe in the range of 1001 to 2024 **(3c),** this indicates that the allele of the SNP was T, for example. When a 1st SNP ID code is decoded on the same joined probe in the range of 3001 to 4024, this indicates that the allele of the SNP was C, for example.

| figure ref. | name | range of codes | example | meaning / significance |
|---|---|---|---|---|
| **2c** (LSO) | 2nd SNP ID | 5001 - 6024 | | identifies SNP |
| | | | 5001 | SNP 1 |
| | | | 5002 | SNP 2 |
| | | | 5003 | SNP 3 |
| | | | 6024 | SNP 1024 |
| **1c** (ASO1) | 1st SNP ID, allele T | 1001 - 2024 | | allele T for SNPs 1 to 1024 |
| | | | 1001 | SNP 1, allele T |
| | | | 1002 | SNP 2, allele T |
| | | | 1003 | SNP 3, allele T |
| | | | 2024 | SNP 1024, allele T |
| | | | | |
| **3c** (ASO2 | 1st SNP ID, allele C | 3001 - 4024 | | allele C for SNPs 1 to 1024 |
| | | | 3001 | SNP 1, allele C |
| | | | 3002 | SNP 2, allele C |
| | | | 3003 | SNP 3, allele C |
| | | | 4024 | SNP 1024, allele C |

It will be observed that at least two codes must be decoded to determine the SNP and its allele. Under this scheme, decoding of a 2nd SNP ID 5002 on a joined probe limits the possible 1 st SNP IDs to either 1002 (from a matching ASO1 to indicate allele T) or 3002 (from a matching ASO2 to indicate allele C). Should 5002 and 1001 be decoded from the same joined probe, however, this indicates that an LSO was not properly matched with the correct ASO1 or ASO2, and the information obtained from this joined probe may not be reliable and may be discarded, depending on the design of the assay. Similarly, decoding 5001 and 3002 would be a mismatch. In another example, detection of only one code would indicate that the probes were not properly joined, or that the decoding was not successful. Other errors can be detected by the decoding of unexpected code combinations, such as primer-dimers, non-specific binding or ligation, and cross-over during PCR applications. Accordingly, the invention provides internal error-detection features that improve the confidence in the resulting data.

### - sample identity

The identity of the sample can be present in one or both Sample ID codes. In one scheme, the 1st and 2nd Sample ID codes on the probes are the same for each sample, so that a resulting joined probe contains redundant Sample IDs. Thus, a joined probe determined to have mismatched Sample ID codes will indicate an incorrectly joined probes, and the results can be disregarded or discarded.

Under a more sophisticated scheme, identity information for greater numbers of samples is provided by combining the information from the 1st sample ID code (appearing as **1b** on ASO1s and as **3b** on ASO2s, depending on which allele is present) and from the 2nd sample ID code (appearing as **2b** on LSOs). The 1st sample ID code (either **1b** or **3b)** has a range of 7001 to 7096. The 1st sample ID code (either **1b** or **3b)** has a range of 8001 to 8384.

| 1st sample ID code **(1b** or **3b)** range from 7001 to 7096 | 2nd sample ID code **(2b)** range from 8001 to 8384 | corresponding sample identity |
|---|---|---|
| 7001 | 8001 | sample 1 |
| 7002 | 8001 | sample 2 |
| 7003 | 8001 | sample 3 |
| ... | ... | ... |
| 7096 | 8001 | sample 96 |
| 7001 | 8002 | sample 97 |
| 7002 | 8002 | sample 98 |
| ... | ... | ... |
| 7096 | 8002 | sample 192 |
| 7001 | 8003 | sample 193 |
| ... | ... | ... |
| 7096 | 8384 | sample 36864 |

This scheme can be summarized as follows: sample number = ((1st sample ID code) - 7000) + 96 x (2nd sample ID code) - 8001). In short, the identity of the sample ID can be deconvoluted from the 1st and 2nd sample ID codes by any number of mathematical encoding schemes.

Accordingly, the invention provides a way to extract all information contained in the joined probe (e.g. SNP, allele, and sample) by decoding only the identification codes. In this way, the identification probes serve as proxies for the complete hybridization sequences of the joined probe. This is particularly suited when paired-end sequencing is used because the codes in some embodiments can be located near or at the 5' and 3' termini.

The invention also provides cost reduction because of the ability to pool joined probes following the initial assay into a high-throughput assay system, while preserving the identity of the sample having the sequence of interest, thus requiring decreased amounts of resources such as reagents, equipment expenses, and time per sample assayed.

The methods described herein for detecting a plurality of sequences of interest in a plurality of samples can be used to evaluate the presence of nucleic acids of particular species. For instance, the methods can be used to detect the presence of pathogenic bacteria in a sample of gut flora. Another example is detecting the species or geographical origin of food species, such as fish or shellfish, to determine safety or detect the substitution of an ersatz foodstuff. Yet another example is detecting the presence of indicator species in an environmental sample to assess the health of an ecosystem. Other uses include detection of a transposon in a functional gene that leads to a disease state, such as cancer, and other mobile genetic elements. Methods of the present invention can be used to measure expression levels of many genes across many samples using a similar analysis of SNPs as described herein. The methods of the present invention can also be used to measure methylation levels of many genes/genomic regions across many samples, where the assaying probes can be designed to interrogate CpG site/CpG islands after bisulfite conversion. Finally, methods of the present invention can also be used to monitor alternative splicing, where the upstream and downstream assay oligonucleotide probes are designed to target adjacent exons or regions that span an exon junction.

The sequences of the first and second sample identification codes can be used not only as identifiers, but also to measure expression abundance. Methods of the present invention allow such measurements simultaneously with many genes across many samples. Alternatively, this first portion of the second sample sequence can be used in code redundancy, and/or as a means to verify a proper match for the extended probe, or to assess the accuracy of the system. As described herein, if the sequence information of the SNP or gene of interest does not match, then the data for this amplified probe is discarded. The tagging information also allows confirmation of the presence of multiple nucleotides in a sequence, such as those described above.

### Kits

The application further relates to a kit for determining the presence of a plurality of nucleotide sequences of interest in a plurality of samples while preserving the identity of each sample. Any of the components or articles used in performing the methods of the invention can be usefully packaged into a kit.

For example, the kits can be packed to include some, many or all of the components or articles used in performing the methods of the invention. Exemplary components include, for example, probes described herein attached to a solid support, hybridization reagents, synthesis reagents for extension and/or ligation of nucleic acids or probes described herein, detection reagents including decoding oligonucleotides. Any of such reagents can include, for example, some, many or all of the buffers, components and/or articles used for performing one or more of the subsequent steps for analysis of a representative sample.

One or more ancillary reagents also can be included in the kits.
Such ancillary reagents can include any of the reagents exemplified above and/or other types of reagents useful in performing the methods of the invention or useful in analysis of a representative sample.

In one embodiment, the kit includes a first and second plurality of probe sets, wherein each probe set includes a first probe having a first identification sequence and first hybridization sequence complementary to a first portion of a sequence of interest, and a second probe having a second identification sequence and a second hybridization sequence complementary to a second portion of the same sequence of interest. In some aspects, the probe sets of the first plurality share a common first sample identification sequence and the probe set of the second plurality share a common second sample identification sequence.

In another embodiment, the probes provided in the kit also include a universal primer sequence. In a further aspect, the invention makes use of a substrate or a solid support including oligonucleotide sequences complementary to one or more of the universal primer sequences. Examples of such substrates or solid support are described herein.

Instructions can further be included in a kit. The instructions can include, for example, procedures for making any components or articles used in the methods of the invention, performing any embodiment of the methods of the invention and/or instructions for performing any of the subsequent analysis and/or decoding steps employing a representative sample.

Software may also be included in the kit (or provided separately) that automates one or more of the steps of the method. For example, decoding step (c) and determining step (d) are particularly suited to being performed on a computer to speed calculation and store the information obtained from performing the method.

The brief section headings and subheadings are for convenience only, and are not intended to define the invention nor limit the scope of the disclosure under those headings. To provide context and to describe the state of the art, this application refers to various publications.

## Claims

1. A method for determining the presence of a plurality of nucleotide sequences of interest in a plurality of samples while preserving the identity of each sample, comprising the steps of:
(a) contacting each sample with a plurality of probe sets, each probe set comprising:
a first probe having a first identification sequence and a first hybridization sequence complementary to a first portion of a sequence of interest, and
a second probe having a second identification sequence and a second hybridization sequence complementary to a second portion of the same sequence of interest;
wherein each of the first and the second identification sequence comprises a separate subsequence for identifying the sample and a separate subsequence for identifying the sequence of interest;
(b) joining the first and second probes of a probe set if the first hybridization sequence is hybridized to the first portion of the sequence of interest, and the second hybridization sequence is hybridized to the second portion of the same sequence of interest;
(c) decoding the identification sequences for a plurality of joined probes, wherein decoding the identification sequences comprises paired-end sequencing of the first and second identification sequences and whereby the identity of each sample is preserved; and
(d) determining the presence of the sequence of interest and the identity of the sample containing the sequence of interest based on identification sequence codes present in each joined probe, wherein said determining comprises an error detection step of:
(i) detecting joint probes having mismatched or unexpected combinations of first and second identification sequence codes; and
(ii) discarding the information obtained from those joined probes having mismatched or unexpected combinations of first and second identification sequence codes.

2. The method of claim 1, wherein the sequences of interest comprise at least one methylation polymorphism.

3. The method of claim 1, wherein the first or the second identification sequence is a single undivided identification sequence.

4. The method of claim 1, wherein the probe set further comprises a third probe having a third identification sequence and a third hybridization sequence complementary to an alternate allele of the sequence of interest.

5. The method of claim 1, wherein joining the first and second probes of at least one probe set further comprises extending the first probe with a polymerase and ligating the extended first probe to the second probe.

6. The method of claim 1, wherein the first probe further comprises a first universal primer sequence and the second probe further comprises a second universal primer sequence.

7. The method of claim 6, further comprising the step of hybridizing the joined probe to a substrate comprising oligonucleotide sequences complementary to the first universal primer sequence.

8. The method of claim 7, wherein the substrate further comprises oligonucleotide sequences complementary to the second universal primer sequence.

9. The method of claim 1, further comprising combining the joined probes of step (b) to form a plurality of joined probe sets from a plurality of samples.

## Patentansprüche

1. Verfahren zur Bestimmung des Vorliegens einer Vielzahl von Nukleotidsequenzen von Interesse in einer Vielzahl von Proben, während die Identität von jeder Probe erhalten bleibt, umfassend die Schritte von:
(a) Inkontaktbringen jeder Probe mit einer Vielzahl von Sondensätzen, wobei jeder Sondensatz Folgendes umfasst:
eine erste Sonde mit einer ersten Identifikationssequenz und einer ersten Hybridisierungssequenz, komplementär zu einem ersten Teil einer Sequenz von Interesse, und
eine zweite Sonde mit einer zweiten Identifikationssequenz und einer zweiten Hybridisierungssequenz, komplementär zu einem zweiten Teil der gleichen Sequenz von Interesse;
wobei jede von der ersten und der zweiten Identifikationssequenz eine separate Subsequenz zum Identifizieren der Probe und eine separate Subsequenz zum Identifizieren der Sequenz von Interesse umfasst;
(b) Verbinden der ersten und zweiten Sonden eines Sondensatzes, wenn die erste Hybridisierungssequenz an den ersten Teil der Sequenz von Interesse hybridisiert wird, und die zweite Hybridisierungssequenz an den zweiten Teil der gleichen Sequenz von Interesse hybridisiert wird;
(c) Dekodieren der Identifikationssequenzen für eine Vielzahl von verbundenen Sonden, wobei das Dekodieren der Identifikationsequenzen die Sequenzierung mit gepaarten Enden von den ersten und zweiten Identifikationssequenzen umfasst und wobei die Identität von jeder Probe erhalten bleibt; und
(d) Bestimmen des Vorliegens der Sequenz von Interesse und der Identität der Probe, enthaltend die Sequenz von Interesse bezogen auf die in jeder verbundenen Sonde vorliegenden Identifikationssequenz-Codes, wobei das Bestimmen einen Fehlerdetektionsschritt wie folgt umfasst:
(i) Detektieren verbundener Sonden mit nicht übereinstimmenden oder unerwarteten Kombinationen von ersten und zweiten Identifikationssequenz-Codes; und
(ii) Verwerfen der aus diesen verbundenen Sonden mit nicht übereinstimmenden oder unerwarteten Kombinationen von ersten und zweiten Identifikationssequenz-Codes erhaltenen Informationen.

2. Verfahren nach Anspruch 1, wobei die Sequenzen von Interesse mindestens einen Methylierungspolymorphismus umfassen.

3. Verfahren nach Anspruch 1, wobei die erste oder die zweite Identifikationssequenz eine einzelne ungeteilte Identifikationssequenz ist.

4. Verfahren nach Anspruch 1, wobei der Sondensatz weiter eine dritte Sonde mit einer dritten Identifikationssequenz und einer dritten Hybridisierungssequenz, komplementär zu einem alternierenden Allel von der Sequenz von Interesse, umfasst.

5. Verfahren nach Anspruch 1, wobei das Verbinden der ersten und zweiten Sonden von mindestens einem Sondensatz weiter das Verlängern der ersten Sonde mit einer Polymerase und Ligieren der verlängerten ersten Sonde an die zweite Sonde umfasst.

6. Verfahren nach Anspruch 1, wobei die erste Sonde weiter eine erste Universal-Primersequenz umfasst und die zweite Sonde weiter eine zweite Universal-Primersequenz umfasst.

7. Verfahren nach Anspruch 6, weiter umfassend den Schritt des Hybridisierens der verbundenen Sonde an ein Substrat, umfassend Oligonukleotidsequenzen, komplementär zur ersten Universal-Primersequenz.

8. Verfahren nach Anspruch 7, wobei das Substrat weiter Oligonukleotidsequenzen, komplementär zur zweiten Universal-Primersequenz, umfasst.

9. Verfahren nach Anspruch 1, weiter umfassend das Kombinieren der verbundenen Sonden von Schritt (b) zum Bilden einer Vielzahl von verbundenen Sondensätzen aus einer Vielzahl von Proben.

## Revendications

1. Procédé permettant de déterminer la présence d'une pluralité de séquences nucléotidiques présentant de l'intérêt dans une pluralité d'échantillons tout en préservant l'identité de chaque échantillon, comprenant les étapes de :
(a) mise en contact de chaque échantillon avec une pluralité d'ensembles de sondes, chaque ensemble de sondes comprenant :
une première sonde ayant une première séquence d'identification et une première séquence d'hybridation complémentaire à une première partie d'une séquence présentant de l'intérêt, et
une seconde sonde ayant une seconde séquence d'identification et une seconde séquence d'hybridation complémentaire à une seconde partie de la même séquence présentant de l'intérêt ;
chacune des première et seconde séquences d'identification comprenant une sous-séquence séparée pour l'identification de l'échantillon et une sous-séquence séparée pour l'identification de la séquence présentant de l'intérêt ;
(b) liaison des première et seconde sondes d'un ensemble de sondes si la première séquence d'hybridation est hybridée à la première partie de la séquence présentant de l'intérêt et la seconde séquence d'hybridation est hybridée à la seconde partie de la même séquence présentant de l'intérêt ;
(c) décodage des séquences d'identification pour une pluralité de sondes liées, le décodage des séquences d'identification comprenant le séquençage d'extrémités appariées des première et seconde séquences d'identification et l'identité de chaque échantillon étant de cette manière préservée ; et
(d) détermination de la présence de la séquence présentant de l'intérêt et de l'identité de l'échantillon contenant la séquence présentant de l'intérêt sur la base des codes de séquence d'identification présents dans chaque sonde liée, ladite détermination comprenant une étape de détection d'erreur consistant à :
(i) détecter des sondes liées ayant des associations mésappariées ou inattendues de premier et second codes de séquence d'identification ; et
(ii) rejeter les informations obtenues à partir de ces sondes liées ayant des associations mésappariées ou inattendues de premier et second codes de séquence d'identification.

2. Procédé selon la revendication 1, les séquences présentant de l'intérêt comprenant au moins un polymorphisme de méthylation.

3. Procédé selon la revendication 1, la première ou la seconde séquence d'identification étant une seule séquence d'identification non divisée.

4. Procédé selon la revendication 1, l'ensemble de sondes comprenant en outre une troisième sonde ayant une troisième séquence d'identification et une troisième séquence d'hybridation complémentaire à un autre allèle de la séquence présentant de l'intérêt.

5. Procédé selon la revendication 1, la liaison des première et seconde sondes d'au moins un ensemble de sondes comprenant en outre l'allongement de la première sonde avec une polymérase et la ligature de la première sonde allongée à la seconde sonde.

6. Procédé selon la revendication 1, la première sonde comprenant en outre une première séquence amorce universelle et la seconde sonde comprenant en outre une seconde séquence amorce universelle.

7. Procédé selon la revendication 6, comprenant en outre l'étape d'hybridation de la sonde liée à un substrat comprenant des séquences oligonucléotidiques complémentaires à la première séquence amorce universelle.

8. Procédé selon la revendication 7, le substrat comprenant en outre des séquences oligonucléotidiques complémentaires à la seconde séquence amorce universelle.

9. Procédé selon la revendication 1, comprenant en outre la liaison des sondes liées de l'étape (b) pour former une pluralité d'ensemble de sondes liées provenant d'une pluralité d'échantillons.
